# EUROPEAN PATENT SPECIFICATION

(11) **EP 4 081 267 B1**
(45) Date of publication and mention of the grant of the patent: **24.04.2024**
(21) Application number: 20842232.9
(22) Date of filing: 28.12.2020
(51) Int. Cl.: A61L 9/12

(54) **DEVICE FOR THE DIFFUSION OF VOLATILE SUBSTANCES**
VORRICHTUNG FÜR DIE DIFFUSION VON FLÜCHTIGEN SUBSTANZEN
DISPOSITIF POUR LA DIFFUSION DE SUBSTANCES VOLATILES

(30) Priority: 27.12.2019 ES 201931158
(43) Date of publication of application: 02.11.2022
(73) Proprietor: Zobele Holding SpA, 38100 Trento (IT)
(72) Inventor: DOYLE, Dominic, 08019 BARCELONA (ES); CAMARERO DÍEZ, Roberto, 08019 BARCELONA (ES); ALFONSO GALLEGO, Fernando, 08019 BARCELONA (ES)
(74) Representative: Herrero & Asociados, S.L.
(86) International application number: PCT/EP2020/087915
(87) International publication number: WO 2021/130386

(56) References cited:
- WO-A1-2006/061803
- WO-A1-2010/019505
- ES-T3- 2 378 509
- GB-A- 2 181 649
- US-A- 5 091 107
- US-A1- 2004 065 749
- US-B2- 7 441 755

## Description

### Object of the invention

The present invention falls within the technical field of air disinfection, sterilisation or deodorisation apparatus, more specifically those using substances that can be evaporated into the air, and concerns in particular a device for the diffusion of volatile substances, in which all its constituent elements are made of the same plastic material.

This feature of the device represents an advantage in terms of environmental sustainability, since the container, once discarded, does not need to be dismantled for efficient separation and recycling of each of its constituent elements. As these are made of the same and unique plastic material, the end user can deposit the used device directly into a plastic container, of those normally used for separating recyclable waste.

### Background to the invention

Within the technical field of diffusion devices for volatile substances, a first distinction should be made between those made of glass type materials and those made of plastics.

Glass containers containing volatile substances, such as liquid fragrances or insecticide compositions, can be recycled for the most part. However, these containers necessarily incorporate capillary type elements to allow the diffusion of the liquid contained to the external environment. These diffusing elements are usually linked to the body of the container in such a way as to ensure that the liquid is not accessible to a user, resulting in a container composed of at least two very different materials, which are difficult to separate from each other and therefore impossible to recycle in a proper way, thus contaminating the final recycled material or being simply disposed of in landfills.

The components of the plastic containers can also be recycled for the most part. However, similarly to glass containers, their final assembly is the result of a mixture of different plastics, which normally cannot be easily separated at the time of disposal. Even if the components can be dismantled, it is a nuisance for the end user, which discourages proper separation and recycling.

Various patent documents relating to containers for volatile releasable substances are known in the current state of the art, each of which has a wide variety of advantageous characteristics and functionalities.

Most devices for the diffusion of volatile substances differ from each other in a variety of functionalities, without special attention having been paid so far to the recyclability of their components.

GB2181649A discloses an air freshener dispenser comprising a necked container, a wick provided in the neck, an emanator pad holder retaining within a containment enclosure an absorbent pad in contact with the wick, and a cover member including at least one fragrance-diffusion aperture.

US5091107A discloses a container provided with an insert member adapted to carry a wick. The insert member is constructed with external neck threads so that it can be releasably threaded into the container in mesh with the internal neck container threads.

US2004065749A1 discloses a composite wick for delivering two liquids with different properties to a surface includes a first section provided for delivering a first liquid to the surface of the wick and a second section provided for delivering a second liquid to the surface of the wick.

WO2006061803A1 discloses a device comprising an active liquid, a reservoir holding the active liquid, a wick/emanator superstructure composed of a wicking part and an emitting part, the latter having an evaporative surface to be directly exposed to the surrounding space when the device is activated and being housed in a moveable housing assembly, activation of the device occurring without need to removing the housing assembly and or the wick/emanator superstructure.

US7441755B2 discloses a device including an active liquid having a specific volatility, a reservoir holding a part of the active liquid, a lid and a wick-emanator superstructure having a wicking part and an emitting part, the latter having an evaporative surface directly exposed to the surrounding space, and a specific absorbency and weight per unit of the evaporative surface.

WO2010019505A1 discloses a device including a wick assembly including at least a wick of porous material capable of being disposed in the opening of the vessel for at least partial submergence in the liquid solution.

ES2378509T3 discloses a refill for a volatile material including a container having a reservoir portion and a neck portion, and a porous wick including an upper portion extending outside the container and a lower portion disposed within the reservoir portion.

There is therefore a need for devices for the diffusion of volatile substances which allow for the separation and recycling of their constituent elements in an effective and simple manner. It is also desirable that such containers should meet the necessary conditions of safety and tightness, and that they should be of simple and economical construction.

### Description of the invention

The object of the invention consists of a device for the diffusion of volatile substances in accordance with Claim 1, in particular, a device for the diffusion of volatile substances comprising a porous and capillary element allowing the substances to be diffused into the surrounding air. All the materials making up the device are made of a single type of plastic, thus providing more efficient recyclability in accordance with currently existing methods. Thus, once the liquid contained in the container has been consumed, the user does not need to dismantle it to separate its components for recycling, but simply places it in one of the containers provided for this purpose.

The device comprises a main container for housing volatile substances (whether liquid fragrances or insecticide compositions), to which a closing lid is attached, equipped with a slot through which a diffusing wick is passed. The lid is covered by a cap that prevents the diffusion of the impregnated wick during transport, storage or any period when the product is not in use.

Other characteristics options are defined in the dependent claims.

In a first preferred embodiment, the components of the device are materialized in polypropylene (PP), while in a second preferred embodiment, the plastic used for the materialization of these components is polyethylene (PE).

The main novelty of the container is that the wick material, which is the aforementioned porous and capillary element that allows the substance to diffuse into the surrounding air, is made of a plastic material similar to that of the other components, thus facilitating its recycling. The plastic used for the wick ensures the necessary functional properties of capillary diffusion.

On the other hand, the groove through which the wick passes usually also includes an adjustment and sealing element, which helps to prevent the liquid contained in the container from spilling through the groove, and also serves to hold the wick in the groove to prevent it from being removed and possible leaks. This adjustment element, due to the functional properties it must have (certain elasticity and flexibility, adjustment capacity), is usually made of plastics that are very different from those used in the rest of the device.

Since the wick of the device described here can be produced by plastic moulding in a three-dimensional shape, or by cutting in a two-dimensional shape, the presence of the above-mentioned adjustment and sealing element is not necessary, as its perfect fit can be ensured by the grooving of the closing cap. In this way, the design and use of materials in the device can be simplified, contributing to easier and more homogeneous recycling.

The device for diffusing volatile substances described above can be connected to an external diffusion device or can be used independently, as it has its own diffusion media.

### Description of the drawings

To complement the description being made and in order to help a better understanding of the characteristics of the invention, in accordance with a preferential example of practical implementation of the same, is accompanied as an integral part of that description a set of drawings where, for illustrative purposes and not limited, the following has been represented:

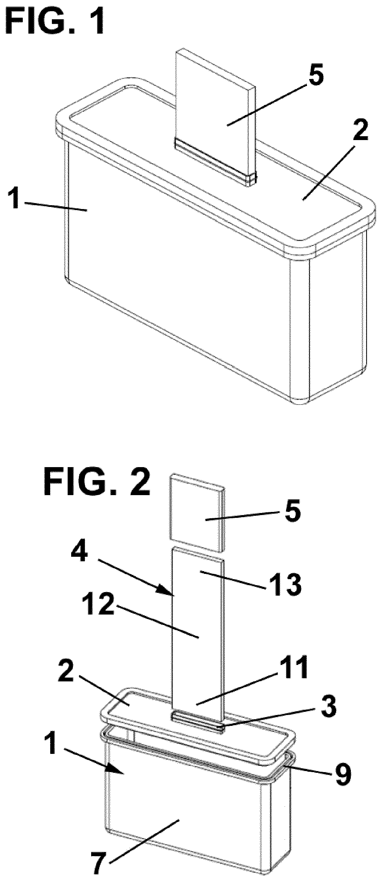

Figure 1 Shows a perspective view of the device for diffusion of volatile substances according to the present invention.
Figure 2.- Shows an exploded view of the device, in which its main constituent elements can be seen.
Figure 3.1.- Shows a front view of the device.
Figure 3.2.- Shows a first side view of the device.
Figure 3.3.- Shows a side view of the device, sectioned according to the imaginary line III-III in figure 3.1.
Figure 4.1.- Shows a second side view of the device.
Figure 4.2.- Shows a front view of the device, sectioned according to the imaginary line IV-IV in figure 4.1.

### Preferred embodiment of the invention

A detailed explanation of an example of a preferred embodiment of the object of the present invention is given below with the help of the above-mentioned figures.

The device for diffusing volatile substances described, shown in the enclosed figures, comprises a container (1), a lid (2) for closing the container (1), equipped with a slot (3), and a diffusion wick (4) that can be fitted into the slot (3), which is partially covered by a cap (5).

The container (1) is made up of a hollow prismatic body, which has an interior housing (6) delimited by lateral walls (7) and a closed lower base (8), and an upper opening (9). The housing (6) is sized and configured to contain a volume of a volatile substance, usually in liquid form and consisting of a fragrance or an insecticide composition. This container (1) is made of a plastic material, which in this case is polypropylene.

The lid (2) of the container (1) has a geometry and dimensions corresponding to those of the opening (9) which it covers, as well as including couplings (10) for physical coupling with the body of the container (1). As indicated above, this lid (2), which is made of a plastic material, preferably polypropylene, has a longitudinal slot (3) for connecting the inner housing (6) with the outer medium to the device.

The diffuser wick (4) can be fitted transversely in the groove (3) to allow controlled release of the volatile substance from inside the housing (6). To this end, the diffuser wick (4) is made up of a body of porous plastic material that allows capillary flow through it. In this preferred embodiment, the body is laminated and has a rectangular geometry, and the plastic in which it is made is sintered polypropylene.

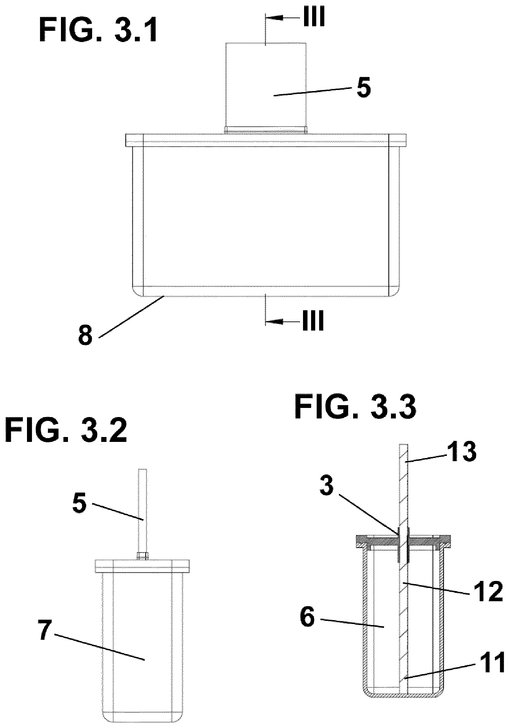

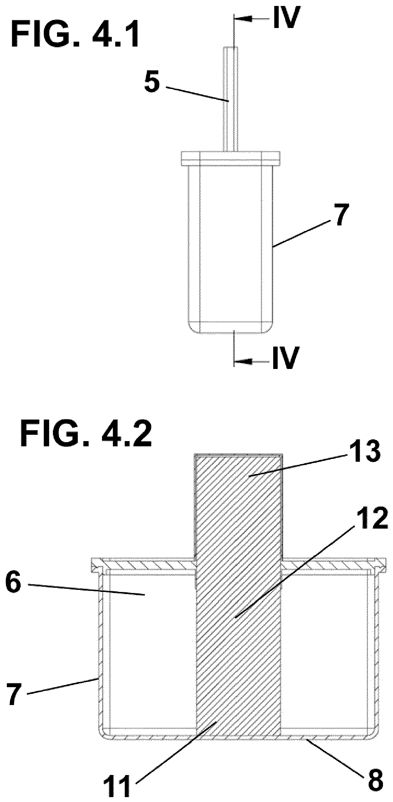

Thus, the diffuser wick (4) has a first end (11), which can be turned towards the housing (6), a central sector (12) which can be fitted into the slot (3), and a second end (13), which can be turned outwards. The volatile substance inside the housing (6) impregnates the first end (11) of the diffuser wick (4), with which it is in direct contact, and flows upwards by capillary action to the central sector (12) and finally to the second end (13), through which it is released by volatilisation into the surrounding environment, as can be seen in figures 3.3 and 4.2.

The cap (5) consists of a plastic part, preferably made of polypropylene, configured and sized for coupling and covering the second end (13) of the diffuser wick (4), to prevent the release of the volatile substance by volatilisation during transport and storage of the device, or during any period when it is desired to prevent such release.

In the first preferred embodiment that is being described, in which all the components are made of polypropylene, they are assembled solidly and mutually by means of ultrasonic welding.

In a second preferred embodiment, in which the assembly between the components of the device is carried out by physical means, this device incorporates at least one adjustment and sealing element, not shown in the attached figures, which ensures correct coupling of the diffuser wick (4) in the slot (3) and prevents possible leaks and spillage of the contents of the housing (6) of the container (1).

An alternative design is also envisaged in which the diffuser wick itself (4) incorporates additional elements for coupling with the groove (3), thus avoiding the need for the adjustment and sealing element. In this alternative embodiment, in which the diffuser wick (4) is moulded, the additional coupling elements consist of a defined extension in the central sector (12) for locking and sealing inside the slot (3).

## Claims

1. Device for the diffusion of volatile substances, comprising:
- a container (1), which holds a liquid with the volatile substances, and which includes an opening (9), for communication from the inside of a housing (6) of the container (1) to the outside;
- a lid (2) that can be attached to the container (1) to close the opening (9);
- a diffuser wick (4) for controlled diffusion of volatile substances, one end (13) of which protrudes from the lid (2); and
- a cap (5) covering the end (13) of the diffuser wick (4) which protrudes from the lid (2);
**characterised in that** the container (1), the lid (2), the diffuser wick (4) and the cap (5) are all made of the same plastic material.

2. Device for the diffusion of volatile substances according to claim 1, wherein the plastic material is polypropylene.

3. Device for the diffusion of volatile substances according to claim 1, wherein the plastic material is polyethylene.

4. Device for the diffusion of volatile substances according to claim 1, wherein the lid (2) comprises a slot (3) through which the diffusion wick (4) is placed.

5. Diffusion device for volatile substances according to claim 1, wherein the diffusion wick (4) comprises a seal extension.

6. Diffusion device for volatile substances according to claim 5, wherein the seal extension is defined in a central sector (12) of the diffusion wick (4) that can be fitted into this slot (3).

## Patentansprüche

1. Vorrichtung für die Diffusion von flüchtigen Substanzen, umfassend:
- einen Behälter (1), der eine Flüssigkeit mit den flüchtigen Substanzen fasst, und der eine Öffnung (9) zur Verbindung vom Inneren eines Gehäuses (6) des Behälters (1) nach außen beinhaltet;
- einen Deckel (2), der am Behälter (1) befestigt sein kann, um die Öffnung (9) zu schließen;
- einen Diffusordocht (4) zur kontrollierten Diffusion von flüchtigen Substanzen, dessen eines Ende (13) von dem Deckel (2) vorsteht; und
- eine Abdeckung (5), die das Ende (13) des Diffusordochts (4) abdeckt, das von dem Deckel (2) vorsteht;
**dadurch gekennzeichnet, dass** der Behälter (1), der Deckel (2), der Diffusordocht (4) und die Abdeckung (5) alle aus demselben Kunststoffmaterial hergestellt sind.

2. Vorrichtung für die Diffusion von flüchtigen Substanzen nach Anspruch 1, wobei das Kunststoffmaterial Polypropylen ist.

3. Vorrichtung für die Diffusion von flüchtigen Substanzen nach Anspruch 1, wobei das Kunststoffmaterial Polyethylen ist.

4. Vorrichtung für die Diffusion von flüchtigen Substanzen nach Anspruch 1, wobei der Deckel (2) einen Schlitz (3) umfasst, durch den der Diffusordocht (4) platziert ist.

5. Diffusionsvorrichtung für flüchtige Substanzen nach Anspruch 1, wobei der Diffusordocht (4) eine Dichtungserweiterung umfasst.

6. Diffusionsvorrichtung für flüchtige Substanzen nach Anspruch 5, wobei die Dichtungserweiterung in einem zentralen Sektor (12) des Diffusordochts (4) festgelegt ist, der in diesen Schlitz (3) eingepasst werden kann.

## Revendications

1. Dispositif de diffusion de substances volatiles, comprenant :
- un récipient (1), qui contient un liquide ayant les substances volatiles et qui a une ouverture (9) de communication de l'intérieur d'un boîtier (6) du récipient (1) à l'extérieur ;
- un couvercle (2) qui peut être fixé au récipient (1) pour fermer l'ouverture (9) ;
- une mèche (4) de diffusion pour une diffusion commandée de substances volatiles, dont l'une des extrémités (13) fait saillie du couvercle (2) ; et
- un capuchon (5) recouvrant l'extrémité (13) de la mèche (4) de diffusion qui fait saillie du couvercle (2) ;
**caractérisé en ce que** le récipient (1), le couvercle (2), la mèche (4) de diffusion et le capuchon (5) sont tous faits en la même matière plastique.

2. Dispositif de diffusion de substances volatiles suivant la revendication 1, dans lequel la matière plastique est le polypropylène.

3. Dispositif de diffusion de substances volatiles suivant la revendication 1, dans lequel la matière plastique est le polyéthylène.

4. Dispositif de diffusion de substances volatiles suivant la revendication 1, dans lequel le couvercle (2) comprend une fente (3) dans laquelle est placée la mèche (4) de diffusion.

5. Dispositif de diffusion de substances volatiles suivant la revendication 1, dans lequel la mèche (4) de diffusion comprend une extension formant joint.

6. Dispositif de diffusion de substances volatiles suivant la revendication 5, dans lequel l'extension formant joint est définie dans un secteur (12) central de la mèche (4) de diffusion, qui peut être adapté dans cette fente (3).
